# EUROPÄISCHE PATENTSCHRIFT

(11) **EP 3 110 392 B1**
(45) Veröffentlichungstag und Bekanntmachung des Hinweises auf die Patenterteilung: **25.11.2020**
(21) Anmeldenummer: 15708139.9
(22) Anmeldetag: 23.02.2015
(51) Int. Cl.: A61K 8/36, A61K 8/46, A61Q 19/00, A61Q 19/08, A61K 9/00, A61K 47/12, A61K 47/20

(54) **TOPISCHE KOSMETISCHE ODER PHARMAZEUTISCHE ZUSAMMENSETZUNG**
TOPICAL COSMETIC OR PHARMACEUTICAL COMPOSITION
COMPOSITION COSMÉTIQUE OU PHARMACEUTIQUE TOPIQUE

(30) Priorität: 25.02.2014 DE 102014102400
(43) Veröffentlichungstag der Anmeldung: 04.01.2017
(73) Patentinhaber: LVS-Capital GmbH, 74906 Bad Rappenau (DE); Caliebe, Reinhard, 95182 Döhlau Ot Tauperlitz (DE)
(72) Erfinder: CALIEBE, Reinhard, 95182 Döhlau (DE)
(74) Vertreter: Kinnstätter, Klaus
(86) Internationale Anmeldenummer: PCT/EP2015/053704
(87) Internationale Veröffentlichungsnummer: WO 2015/128280

(56) Entgegenhaltungen:
- WO-A2-2010/083035
- CN-A- 1 633 995
- CN-A- 101 700 246
- US-A- 5 552 153
- US-A- 5 916 587
- US-A1- 2007 148 218
- US-A1- 2008 319 092
- US-A1- 2009 215 888
- Anonymous: "Diclofenac Sodium | CAS 15307-79-6 | SCBT", , 13 July 2017 (2017-07-13), XP055390500, Retrieved from the Internet: URL:https://www.scbt.com/scbt/de/product/d iclofenac-sodium-15307-79-6 [retrieved on 2017-07-13]

## Beschreibung

### Gebiet der Erfindung

Die Erfindung betrifft eine topische kosmetische und/oder pharmazeutische Zusammensetzung die aus einer Ölphase besteht, welche Dimethylsulfoxid, eine oder mehrere flüssige Monocarbonsäuren mit mindestens 6 Kohlenstoffatomen und mindestens einen Wirkstoff gelöst enthält.

### Hintergrund der Erfindung

Viele kosmetische oder pharmazeutische Wirkstoffe werden schlecht durch die Haut absorbiert. Dies ist nachteilig, da eine Absorption durch die Haut bei wenig magen- oder darmverträglichen Wirkstoffen oder auch bei Wirkstoffen, die weder im Magen noch im Darm gut absorbiert werden, sehr wünschenswert wäre. Ferner wird bei einer Absorption durch die Haut der sogenannte "First-pass-Effekt" durch die Leber vermieden, durch den viele Wirkstoffe nach der Absorption im Magen oder Darm sofort in der Leber in oft erheblichem Umfang abgebaut werden, was die Verabreichung höherer Dosen erfordert, die den Körper belasten.

Es sind im Stand der Technik zahlreiche Penetrationsverstärker oder Kombinationen von Penetrationsverstärkern bekannt, die die Absorption eines Wirkstoffs aus der wässrigen oder Ölphase einer topischen kosmetischen und/oder pharmazeutischen Formulierung durch die Haut fördern.

Jedoch besteht weiterhin ein großer Bedarf an neuen galenischen Systemen für Wirkstoffe in topischen oder transdermalen kosmetischen oder pharmazeutischen Zusammensetzungen, da es noch viele Wirkstoffe gibt, die bisher nicht in ausreichendem Maße oder gar nicht durch die Haut verabreicht werden können.

Das Ziel der Erfindung war es, ein galenisches System zu finden, das eine Absorption auch von Wirkstoffen, die bisher nicht oder nur schlecht durch die Haut verabreichbar waren, durch die Haut ermöglicht.

Die Präparate der US 5 552 153 A, US 5 916 687 A, US 2009/0215888, WO 2010/083035, CN 101700245 A, CN 1633995 A und US 2008/319092 A enthalten alle Dimethylsulfoxid, Monocarbonsäure und Wirkstoff in einer hydrophilen Phase.

### Zusammenfassung der Erfindung

Die Erfindung betrifft eine topische kosmetische und/oder pharmazeutische Zusammensetzung, bestehend aus einer hydrophobe Ölphase, wobei die hydrophobe Ölphase Dimethylsulfoxid, eine oder mehrere bei Raumtemperatur flüssige Monocarbonsäuren mit mindestens 6 Kohlenstoffatomen und mindestens einen Wirkstoff gelöst enthält, welcher einen ungesättigten und/oder aromatischen Ring und/oder eine Wasserstoffbrücken bildende Gruppe enthält, wobei das Mol-Verhältnis von Wirkstoff(en) : Dimethylsulfoxid : Monocarbonsäure(n) im Bereich von 1 : 1 - 700 : 1- 180 liegt und das Mol-Verhältnis von Dimethylsulfoxid : Monocarbonsäure im Bereich von 1: 5 bis 12: 1 liegt und eine Mischung bestehend aus Dimethylsulfoxid, Monocarbonsäure und Wirkstoff, wie vorstehend definiert, eine klare Phase bilden kann, und wobei die Ölphase weiter mindestens ein Triglycerid oder natürliches Triglyceridöl umfasst.

### Detaillierte Beschreibung

Dimethylsulfoxid (DMSO) ist schon lange als sehr guter Penetrationsverstärker bekannt. Jedoch wurde es bisher zusammen mit einem in wässriger oder zumindest hydrophiler Phase einer topischen kosmetischen oder pharmazeutischen Zusammensetzung vorliegenden Wirkstoff verwendet. Jedoch sind viele Wirkstoffe in wässriger oder Öl-Phase nicht lösbar. Ferner lässt die Penetration aus wässriger oder hydrophiler Phase häufig zu wünschen übrig.

Es wurde nun völlig überraschend entdeckt, dass ein Wirkstoff, der mit einer Kombination von Dimethylsulfoxid und einer flüssigen Monocarbonsäure mit mindestens 6 Kohlenstoffatomen in dem vorstehend angegebenen Verhältnis assoziiert und in einer Ölphase gelöst ist, eine ganz hervorragende Absorption durch die Haut zeigt.

Die Ölphase oder hydrophobe Phase der vorliegenden Erfindung enthält bevorzugt mindestens etwa 10 Gew.-% eines Öls, das bevorzugt aus Ethern, z.B. Dicaprylether, Carbonaten, z.B. Dicaprylcarbonat, Butylenglycolestern, z.B. Buylenglycolcaprylat, Weinsäureestern, z.B. Dialkyltartrat, Succinaten, z.B. Caproyl/Caprinoyldiglyceridsuccinat, Triglyceriden, z.B. Trioleoyltriglycerid, Glycerid-Gemischen, die mindestens 5 Gew.-% Triglyceride enthalten, z.B. Cocoglycerid, Kohlenwasserstoffen (Paraffinöl, Mineralöl, hydriertes Polydecen, Isoeicosan Dioctylcycolhexan, Squalan, Squalen), Silikonölen, z.B. Cyclomethicon (Octamethylcyclotetrasiloxan), Hexamethylcyclotrisiloxan, Polydimethylsiloxan und Poly(methylphenylsiloxan), und Mischungen dieser Öle ausgewählt ist.

Fettsäuretriglyceride, namentlich die Triglycerinester gesättigter und/oder ungesättigter, verzweigter und/oder unverzweigter Alkancarbonsäuren einer Kettenlänge von 8 bis 24, insbesondere 12 bis 18 C-Atomen sind besonders bevorzugt. Diese können in synthetischer oder halbsynthetischer Form oder in natürlicher Form pflanzlicher oder tierischer Fettsäuretriglycerid-Öle, wie z.B. Olivenöl, Sonnenblumenöl, Sojaöl, Erdnussöl, Rapsöl, Sesamöl, Haselnussöl, Mandelöl, Palmöl, Kokosöl, Palmkernöl und/oder Distelöl, eingesetzt werden.

Die Ölphase kann mehr bevorzugt mindestens etwa 20 Gew.-%, z.B. etwa mindestens 30 Gew.-%, etwa mindestens 40 Gew.-%, etwa mindestens 50 Gew.-%, etwa mindestens 60 Gew.-%, etwa mindestens 70 Gew.-%, etwa mindestens 80 Gew.-%, oder mindestens etwa 90 Gew.% eines oder mehrerer der vorgenannten Öle umfassen.

Wachse wie Bienenwachs, Methylpalmitat, Cetylpalmitat, C₂₀₋₄₀-Alkystearat und C₁₈₋₃₆-Säuretriglycerid können ein Bestandteil der Ölphase sein. Ferner kann die Ölphase bis zu etwa 10 oder etwa 20 oder etwa 30 oder etwa 40 Gew.-% eines oder mehrerer Monoglyceride und/oder Diglyceride enthalten. Auch öllösliche Mono-, Di- oder Tricarbonsäuren, die von der oder den flüssigen Monocarbonsäuren mit mindestens 6 Kohlenstoffatomen, die zusammen mit DMSO mit dem Wirkstoff assoziiert sind, verschieden sind, können in der Ölphase enthalten sein.

Die flüssige(n) Monocarbonsäure(n) mit mindestens 6 Kohlenstoffatomen, die zusammen mit DMSO mit dem Wirkstoff assoziiert ist/sind, können aus allen bei Raumtemperatur (25° C) flüssigen, gerad- oder verzweigtkettigen, gesättigten oder ein- oder mehrfach ungesättigten Monocarbonsäuren mit mindestens 6 Kohlenstoffatomen ausgewählt sein, wie Capronsäure, Önanthsäure, Carpylsäure, Pelargonsäure, Linolsäure, Linolensäure, Myristinoleinsäure, Palmitoleinsäure, Elaidinsäure, Ölsäure und Phytansäure. Ölsäure ist besonders bevorzugt.

Das Verhältnis von Wirkstoff: DMSO : flüssiger Carbonsäure mit mindestens 6 C-Atomen liegt im Bereich von etwa 1 : etwa 1 - etwa 700: etwa 1 - etwa 180, bevorzugt von etwa 1: etwa 1 - etwa 350 : etwa 1 - etwa 150, z.B. etwa 1 : etwa 1 - etwa 300: etwa 1 - etwa 130, mit der Maßgabe, dass das Verhältnis von DMSO : flüssiger Carbonsäure mit mindestens 6 C-Atomen im Bereich von etwa 1 : etwa 5 bis etwa 12 : etwa 1, bevorzugt etwa 1 : etwa 2 bis etwa 12 : etwa 1 liegt.

Die Wirkstoffe, die erfindungsgemäß mit DMSO und einer oder mehreren flüssigen Monocarbonsäuren mit mindestens 6 C-Atomen assoziiert in der Ölphase vorliegen können, unterliegen keinerlei Beschränkung, solange sie sich mit den vorstehend angegebenen Verhältnissen von DMSO und der oder den flüssigen Monocarbonsäuren mit mindestens 6 C-Atomen in der Ölphase lösen lassen. Sie können aus allen synthetischen und natürlichen (z.B. pflanzlichen) pharmazeutischen und kosmetischen Wirkstoffen und Wirkstoffklassen ausgewählt sein.

Dazu gehören beispielsweise die folgenden pharmazeutischen Wirkstoffklassen: ACE-Hemmer, Adrenorezeptorenblocker, Sympathomimetika, Anti-Angina pectoris-Mittel, Antiarrhytmica, Thrombozytenaggregationshemmer, Antikoagulantien, Cyclooxygenase-Hemmer, Thromboxansynthase-Hemmer, blutdrucksenkende Mittel, Antirheumatika und nichtsteroidale Antirheumatika, Antianginosa, Lipidsenker, Antidiabetika, entzündungshemmende Mittel, Calciumkanal-Blocker, COX-2-Hemmer, direkte Thrombin-Hemmer, Diuretika, inotrope Mittel, Vasodilatatoren, Vasopressoren, Analgetika, Opioid-Analgetika, Antiasthma-Mittel, Antibiotika, Antivirusmittel, Antiprotozoenmittel, Antidepressiva, Antiepileptika, Neuroleptika, Antimigräne-Mittel, antineoplastische Mittel, Immunsuppressiva, Antitussiva, Anxiolytika, Sedativa, Hypnotika, Stimulanzien, Antiparkinsonmittel, gastrointestinale Mittel, Corticosteroide, Sexualhormone, Nährstoffe (z.B. Vitamine, essentielle Fettsäuren, Aminosäuren), Flavonoide, Isoflavonoide und Carotinoide, Terpene und Terpenoide.

Zu den kosmetischen Wirkstoffen, die in der erfindungsgemäßen Zusammensetzung enthalten sein können, zählen ohne Anspruch auf Vollständigkeit z.B. Antiaknemittel, Mittel gegen Hyperpigmentierung, Mittel gegen Sonnenbrand, Mittel gegen Rosacea, Narbenbehandlungsmittel, Hautpflegemittel und Haarwuchs-fördernde Mittel.

Naturstoffe aus Pflanzen und davon abgeleitete Stoffe sind besonders bevorzugte Wirkstoffe in der erfindungsgemäßen Zusammensetzung. Im Folgenden werden ohne Anspruch auf Vollständigkeit einige Pflanzen und in ihnen enthaltene Wirkstoffe aufgezählt, die in der erfindungsgemäßen Zusammensetzung enthalten sein können.

In Weihrauch *(Boswellia sacra, Boswellia carteri, Boswellia frerena, Boswellia neglecta, Boswellia papyrifera, Boswellia serrata, Boswellia ameero, Boswellia elongata, Boswellia socotrana, Canarium libertianum, Canarium bengalese*) enthaltene Wirkstoffe:
Natürliche Boswelliasäuren, wie 3α-O-Acetyl-11-keto-α-boswelliasäure, 3α-O-Acetyl-11-keto-β-boswelliasäure, 11-Keto-α-boswelliasäure, 11-Keto-β-boswelliasäure, 11α-Hydroxy-β-boswelliasäure, 11β-Hydroxy-β-boswelliasäure, 3α-O-Acetyl-α-boswelliasäure, 3α-O-Acetyl-β-boswelliasäure, 3α-O-Acetyl-11-hydroxy-β-boswelliasäure, α-Boswelliasäure, β-Boswelliasäure, 3β-Hydroxy-β-boswelliasäure, 3α-O-Acetyl-11-hydroxy-β-boswelliasäure, 2-Hydroxy-β-boswelliasäure, 2-Hydroxy-11-keto-β-boswelliasäure, Abbau- und Umwandlungsprodukte der natürlichen Boswelliasäuren, wie 9,11-Dehydro-β-boswelliasäure, 9,11-Dehydro-α-boswelliasäure, 3α-O-Acetyl-9,11-dehydro-α-boswelliasäure, 3α-O-Acetyl-9,11-dehydro-β-boswelliasäure, 3α-O-Acetyl-11-methoxy-β-boswelliasäure, synthetische Derivate der Boswelliasäuren, wie 3-O-Glutaroyl-β-boswelliasäure, 3-O-Glutaroyl-11-keto-β-boswelliasäure, 3-O-Succinoyl-β-boswelliasäure, 3-O-Succinoyl-11-keto-β-boswelliasäure, 3-O-Oxaloyl-β-boswelliasäure, 3-O-Oxaloyl-11-keto-β-boswelliasäure, 3-O-Galloyl-β-boswelliasäure, 3-O-Galloyl-11-keto-β-boswelliasäure, 3-O-Carboxymethyl-β-boswelliasäure, 3-O-Carboxymethyl-11-keto-β-boswelliasäure, 3-O-Carboxymethyl-11-keto-β-boswelliasäurecarboxymethylester, 11-Keto-β-boswelliasäurecarboxymethylester, 11α-Hydroxy-β-boswelliasäure, 11 β-Hydroxy-β-boswelliasäure, Cis-diol-β-boswelliasäure, Malonsäure-di-3-O-β-boswelliasäureester, Malonsäure-di-3-O-11-keto-β-boswelliasäureester, 2,3-Dehydro-β-boswelliasäure, 2,3-Dehydro-11-keto-β-Boswelliasäure, 1-Hydroxy-2,3-dehydro-11-keto-β-boswelliasäure, 1-Keto-2,3-dehydro-11-keto-β-boswelliasäure, 11β-Hydroxy-β-boswelliasäure und 11α-Hydroxy-β-boswelliasäure;
Lupansäuren, wie Lupansäure, 3α-O-Acetyllupansäure, 3α-O-Acetyl-27-hydroxylupansäure und 3α-O-Acetyl-28β-hydroxylupansäure;
Tirucallensäuren, wie 3-Oxotirucallensäure, 3a-Hydroxytirucallensäure, 3β-Hydroxytirucallensäure, 3α-O-Acetyltirucallensäure und 3a-Hydroxy-7,24-dientirucallensäure;
3,4-seco-Triterpensäuren, wie 4(23)-Dihydrorobursäure, 4(23)-Dihydronycthantinsäure, 4(23)-Dihydrocanariensäure, Canariensäure, Robursäure, Ketorobursäure und 4(23)-Dihydroketorobursäure;
Triterpene, wie Protopanaxadiol, 3-Acetoxy-16,20-dihydroxydammaren-24, Lupeol, α-Amyrin, β-Amyrin, α-Amyrenon und β-Amyrenon; sowie
Oleanolsäure und Ursolsäure.

In Gelbwurzel (*Curcuma longa*) enthaltene Wirkstoffe:
Curcumin, Demethoxycurcumin, Bisdesmethoxycurcumin und Calebin-A.

In Primelwurzel (*Primula veris L., Primula elatior*) enthaltene Wirkstoffe:
Protoprimulagenin A, Priverogenins B, Echinocystsäure.

In Broccoli (*Brassica oleracea var. Italica*) enthaltene Wirkstoffe
Sulforaphan (Produkt von Gluccorapahin und Myrosinease in Anwesenheit von Wasser) und Quercetin.

In Kamille (*Matricaria chamomilla*) enthaltene Wirkstoffe:
Quercetin, Bisabolol, (-)-α-Bisabolol, β-Bisabolol, Luteolin, Apigenin, Umbelliferon, Herniarin, Aesculin, Scopoletin, Matricin, Chamazulen, Bisabololoxid A und Flavonoide, wie Chryseriol, Isorhamnetin, 6-Methoxykämpferol, Eupalitin, Patuletin, Axillarin, Spinacetin, Eupatolitin, Chrysosplenol (C - F), Chrysosplenetin und Jaceidin.

In Ringelblume (*Calendula officinalis*) enthaltene Wirkstoffe:
α-Amyrin, β-Amyrin, Lupeol, Taraxasterol, ψ-Taraxasterol, Arnidiol, Heliantriol B0, Heliantriol B1, Lutein, Zeaxanthin, Isorhamnetin, Scopoletin, Aesculetin, Sterole, wie β-Sitosterol, Stigmasterol, Campesterol und Avenasterol, Stanole, wie β-Sitostanol und Cholestanol, Triterpenalkohole, wie Oleanan, Maniladiol, Longispinogenin, Ursan, Brein, Lupan, Lupeol, Calenduladiol, Ursodiol, Erythrodiol, Uvaol, Heliantriol, Heliantriol C und Heliantriol F.

In Beinwell (*Symphytum officinale*) enthaltene Wirkstoffe:
Allantoin, Asparagin und Rosmarinsäure.

In Arnika (*Arnica montana*) enthaltene Wirkstoffe:
Sesquiterpenlactone, Helenalin, 11,12-Dihydrohelenalin, Arnifolin, Chamissonolide und Thymol.

In Baldrian (*Valeriana officinalis*) enthaltene Wirkstoffe:
Valepotriate, Valtrat, Isovaltrat, Didrovaltrat, IVHD-Valtrat, Baldrinal, Valerensäure, Acetoxyvalerensäure und Actinidin.

In Benzoe-Harz (*Styrax officinalis, Styrax tonkinensis, Styrax benzoin Dryand*) enthaltene Wirkstoffe:
α-Siaresinolsäure, Coniferylbenzoat, p-Cumaroylbenzoat, Cinnamoylbenzoat und Benzoesäure.

In Brennnessel (*Urtica dioica, Urtica urens*) enthaltene Wirkstoffe:
Quercetin, Isorhamnetin und Kämpferol.

In Prärie-Igelkopf (*Echinacea pallida*) enthaltene Wirkstoffe:
Cichoriensäure und Diferuloylweinsäure.

In Efeu (*Hedera helix* L.) enthaltene Wirkstoffe:
Oleanolsäure, Hederagenin und Bayogenin.

In Frauenmantel (*Alchemilla vulgaris* L. s. I.) enthaltene Wirkstoffe:
Ellagsäure, Quercetin, Agrimoniin und Ellagitannine.

In Fußblattwurzel / Podophyllwurzelstock (*Podophyllum peltatum* L.) enthaltene Wirkstoffe:
Podophyllotoxin, β-Peltatin, α-Peltatin, 4'-Demethylpodophyllotoxin und Desoxypodophyllotoxin.

In Galgant (*Alpinia officinarum*) enthaltene Wirkstoffe:
Borneol und Acetoxychavicolacetat.

In Gewürznelken (*Syzygium aromaticum*) enthaltene Wirkstoffe:
Maslinsäure, Oleanolsäure, Kämpferol, Kämpferid und Rhamnetin.

In Gipskraut / Weiße Seifenwurzel (*Gypsophila paniculata* L.) enthaltene Wirkstoffe: Gypsogenin und Quillajasäure.

In Mäusedornwurzelstock - Rusci rhizoma (*Ruscus aculeatus* L.) enthaltene Wirkstoffe:
Ruscogenin und Neoruscogenin.

In Melissenblätter (*Melissa officinalis* L.) enthaltene Wirkstoffe:
Citronellal, Geranial, Neral, Geraniol, Geranylacetat, Linalool, Germacren D, Rosmarinsäure, Kaffeesäure, p-Cumarsäure und Ferulasäure.

In Pfefferminzblätter - Menthae piperitae folium (*Mentha x piperita* L.) enthaltene Wirkstoffe:
Menthol, Menthon, Menthylacetat, Menthofuran und Neomenthol.

In Kapland-Pelargonie (*Pelargonium-sidoides*) enthaltene Wirkstoffe:
Umckalin, Umckalin-7-sulfat, Trimethoxycumarin, Scopoletin, 6,8-Dihydroxy-5,7-dimethoxycumarin, 5,6,7,8-Tetramethoxycumarin, 6-Hydroxy-5,7-dimethoxycumarin-8-sulfat.

In Sägepalmfrucht - Sabal serrulatae fructus (*Serenoa repens*) enthaltene Wirkstoffe: β-Sitosterol (das außerdem noch in vielen anderen Pflanzen enthalten ist).

In Grünem Tee (*Camellia sinensis*) enthaltene Wirkstoffe:
Epigallocatechingallat und Coffein.

Im Niembaum (*Azadirachta indica*) enthaltene Wirkstoffe:
Kämpferol, Quercetin, Scopoletin, Myricetin und β-Sitosterol.

In Nussgras-Wurzeln (*Cyperus rotundus* L.) enthaltene Wirkstoffe:
α-Cyperon, β-Selinen,1,8-Cineol, β-Pinen, Flavonoide, Limonen, Oleanolsäure und Polyphenole.

In Zitrone (*Citrus limon*) enthaltene Wirkstoffe:
Zitronensäure, 1,8-Cineole, Essigsäure, Ascorbinsäure, β-Sitosterol, Limonen, Luteolin, p-Cumarsäure und Thymol.

In Essig enthaltener Wirkstoff:
Essigsäure

Von diesen Wirkstoffen sind derzeit α-Amyrin, β-Amyrin, 3α-O-Acetyl-β-boswelliasäure, 3α-O-Acetyl-11-keto-β-boswelliasäure, 3α-O-Acetyl-11-hydroxy-β-boswelliasäure, 3α-O-Acetyl-11-hydroxy-β-boswelliasäure, 3α-O-Acetyl-9,11-dehydro-β-boswelliasäure, 3α-O-Acetyl-11-methoxy-β-boswelliasäure, 3β-Hydroxy-β-boswelliasäure, 11α-Hydroxy-β-boswelliasäure, 11β-Hydroxy-β-boswelliasäure, 11-Keto-ß-boswelliasäure, β-Boswelliasäure, 3-O-Galloyl-β-boswelliasäure, 3-O-Galloyl-11-keto-β-boswelliasäure, Oleanolsäure, Ursolsäure, Maslinsäure, Echinocystsäure, Lupansäure, Ferulasäure, 3α-O-Acetyllupansäure, 3α-O-Acetyl-28β-hydroxylupansäure, Robursäure, 4(23)-Dihydroketorobursäure, Bisabolol, (-)-α-Bisabolol, β-Bisabolol, Sulforaphan, Allantoin, Curcumin, Bisdesmethoxycurcumin, Quercetin, Luteolin, Apigenin, Umckalin, Scopoletin, Helenalin, 11,12-Dihydrohelenalin, Kämpferol, Kämpferid, Rhamnetin, β-Sitosterol, Epigallocatechingallat und Coffein besonders bevorzugt.

Auch natürlich vorkommende Sexualhormone, beispielsweise Progesteron und Testosteron, sind als Wirksubstanzen für die erfindungsgemäße Zusammensetzung bevorzugt.

Als Beispiele für die unzähligen synthetischen Wirkstoffe, die in der erfindungsgemäßen Zusammensetzung enthalten sein können, sollen lediglich Acetylsalicylsäure, Salicylsäure, Ibuprofen, Diclofenac, Diclofenac-Natrium, Ketoprofen, Calcium-D(+)-pantothenat und Pantothensäure, genannt werden.

Die erfindungsgemäßen Zusammensetzungen können als jegliche topische kosmetische oder pharmazeutische Zubereitung vorliegen, die aus einer Ölphasebesteht. Dazu zählen insbesondere Öle und Salben.

Die erfindungsgemäßen kosmetischen und/oder pharmazeutischen topischen Zusammensetzungen können Hilfsstoffe enthalten, wie sie üblicherweise in solchen hydrophoben Zubereitungen verwendet werden, z. B. Konservierungsmittel, Bakterizide, Viruzide, Parfüme, Farbstoffe, Pigmente, die färbende Wirkung haben, UV-Schutzmittel, z. B. Ti02-Lichtschutzpigmente, NO-Synthasehemmer, Verdickungsmittel wie verdickende Polymere, Fette, Öle und Wachse.

Neben dem oder den Wirkstoffen können in der Ölphase auch weitere kosmetisch und/oder pharmazeutisch aktive Substanzen aus allen Wirkstoffklassen vorliegen, wie z.B. Vitamine, Hormone, Steroide, Keratolytika, die Durchblutung fördernde Wirkstoffe, Antimykotika, Antibiotika, Antihistaminika, Antianginosa und Antiphlogistika.

Für die Herstellung der Lösung von Dimethylsulfoxid, einer oder mehrerer flüssiger Fettsäuren mit mindestens 6 Kohlenstoffatomen und eines oder mehrerer Wirkstoffe in der Ölphase wird im Allgemeinen zuerst der oder die Wirkstoff(e) mit einer minimalen Menge DMSO gemischt, bis eine fluide Phase entsteht. Diese wird dann weiter mit einer minimalen Menge an Carbonsäure behandelt, bis sich wiederum eine einzige fluide Phase gebildet hat, die dann in dem Öl gelöst wird.

In manchen Fällen, insbesondere bei Harz- oder Pflanzen-Auszügen, kann es auch vorteilhaft sein, die Wirkstoffe zunächst in Ölsäure zu lösen, die Lösung gegebenenfalls zu filtrieren, dann das DMSO und anschließend das Öl dazuzugeben, um eine klare Lösung von Wirkstoff, DMSO und Carbonsäure im Öl zu erhalten.

Bei einigen anderen, z.B. ionischen, Wirkstoffen ist manchmal auch eine gleichzeitige Zugabe von DMSO und Carbonsäure erforderlich, um eine einzige fluide Phase zu erhalten. Hier kann es auch erforderlich sein, dass weiteres DMSO und weitere Carbonsäure in der Ölphase vorliegen muss, damit sich die fluide Wirkstoff/DMSO/Carbonsäure-Phase beim Mischen mit der Ölphase unter Bildung einer klaren Lösung in der Letztgenannten löst.

Ohne dass man durch eine Theorie beschränkt sein will, wird vermutet, dass der eine oder die mehreren Wirkstoff(e) in der Ölphase zumindest teilweise mit Dimethylsulfoxid (DMSO) und der oder den flüssigen Monocarbonsäuren mit mindestens 6 Kohlenstoffatomen assoziiert vorliegt. Die Ölphase ist, wenn sie lediglich das DMSO, die Monocarbonsäure(n) und den oder die Wirkstoff(e) gelöst enthält, typisch ein klares Fluid.

Es gibt derzeit keine umfassende Theorie, die anhand der Struktur einer chemischen Verbindung zuverlässig voraussagen könnte, ob diese Verbindung wie vorstehend beschrieben in der Ölphase vorliegen kann. Hier ist, wie generell auf dem Gebiet der Löslichkeit von chemischen Verbindungen, mit Routineversuchen zu ermitteln, ob dies für eine spezielle Verbindung der Fall ist. Es kann nur allgemein angegeben werden, dass es scheint, dass chemische Verbindungen, die wie vorstehend angegeben in der Ölphase vorliegen können, einen ungesättigten oder aromatischen Ring und/oder mindestens eine Wasserstoffbrückenbindung-bildende Gruppe aufweisen sollten.

Die folgenden Beispiele erläutern die Erfindung näher.

### Beispiele

### Beispiel 1: Herstellung einer Lösung von mit DMSO und Ölsäure assoziiertem Curcumin in Triolein

### Curcumin

| | |
|---|---|
| Ketoform: | 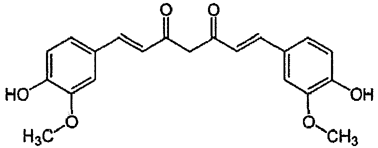 |
| Enolform: | 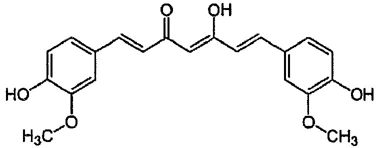 |

0,01 g (0,027 mMol) Curcumin wurden mit 0,1 ml (0,110 g, 1,408 mMol) Dimethylsulfoxid (DMSO) vermischt, geschüttelt und 30 Minuten stehen gelassen. Es bildete sich eine glasklare rote Flüssigkeit. Diese wurde weiter mit 0,17 ml (0,151 g, 0,536 mMol) Ölsäure versetzt, geschüttelt und 10 Minuten stehen gelassen. Es bildete sich eine glasklare gelb-orange flüssige Phase. Die monophasische Curcumin-DMSO-Ölsäure-Assoziation löste sich ohne Rückstand in 1,5 ml (1,365 g, 1,542 mMol) Triolein. Es bildete sich eine glasklare gelbe flüssige Phase.
Mol-Verhältnis Curcumin:DMSO:Ölsäure = 1:51,9:19,7

### Beispiel 2: Herstellung einer Lösung von mit DMSO und Ölsäure assoziierter 3-O-Acetyl-β-Boswelliasäure in Triolein

### 3-O-Acetyl-β-Boswelliasäure

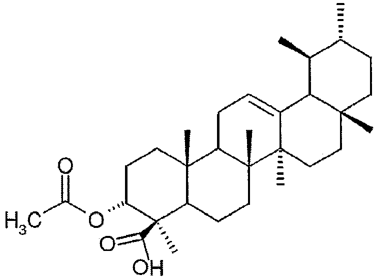

0,002 g (0,004 mMol) 3-O-Acetyl-β-Boswelliasäure wurden mit 0,06 ml (0,066 g, 0,845 mMol) Dimethylsulfoxid (DMSO) vermischt, geschüttelt und 10 Minuten stehen gelassen. Es bildete sich eine glasklare Flüssigkeit. Diese wurde weiter mit 0,06 ml (0,053 g, 0,189 mMol) Ölsäure versetzt, geschüttelt und 10 Minuten stehen gelassen. Es bildete sich eine glasklare flüssige Phase.Die monophasische 3-O-Acetyl-β-Boswelliasäure-DMSO-Ölsäure-Assoziation löste sich ohne Rückstand in 1,0 ml (0,910 g, 1,028 mMol) Triolein. Es bildete sich eine glasklare flüssige Phase.
Mol-Verhältnis 3-O-Acetyl-β-Boswelliasäure:DMSO:Ölsäure = 1:210:47

### Beispiel 3: Herstellung einer Lösung von mit DMSO und Ölsäure assoziiertem Coffein in Triolein

### Coffein

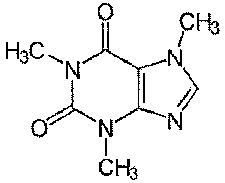

0,004 g (0,021 mMol) Coffein wurden mit 0,41 ml (0,451 g, 5,772 mMol) Dimethylsulfoxid (DMSO) vermischt, geschüttelt und 30 Minuten stehen gelassen. Es bildete sich eine glasklare Flüssigkeit. Diese wurde weiter mit 0,8 ml (0,712 g, 2,521 mMol) Ölsäure versetzt, geschüttelt und 10 Minuten stehen gelassen. Es bildete sich eine glasklare flüssige Phase. Die monophasische Coffein-DMSO-Ölsäure-Assoziation löste sich ohne Rückstand in 1,5 ml (1,365 g, 1,542 mMol) Triolein. Es bildete sich eine glasklare flüssige Phase.
Mol-Verhältnis Coffein:DMSO:Ölsäure = 1:280,2:122,4

### Beispiel 4: Herstellung einer Lösung von mit DMSO und Ölsäure assoziiertem Progesteron in Triolein

### Progesteron

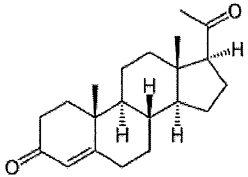

0,06 g (0,191 mMol) Progesteron wurden mit 2,205 ml (2,426 g, 31,044 mMol) Dimethylsulfoxid (DMSO) vermischt, geschüttelt und 30 Minuten stehen gelassen. Es bildete sich eine glasklare Flüssigkeit. Diese wurde weiter mit 1,563 ml (1,391 g, 4,925 mMol) Ölsäure versetzt, geschüttelt und 10 Minuten stehen gelassen. Es bildete sich eine glasklare flüssige Phase. Die monophasische Progesteron-DMSO-Ölsäure-Assoziation löste sich ohne Rückstand in 1,5 ml (1,365 g, 1,542 mMol) Triolein. Es bildete sich eine glasklare flüssige Phase.
Mol-Verhältnis Progesteron:DMSO:Ölsäure = 1:162,7:25,8

### Beispiel 5: Herstellung einer Lösung von mit DMSO und Ölsäure assoziiertem Testosteron in Triolein

### Testosteron

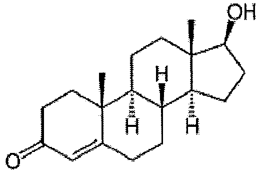

0,06 g (0,208 mMol) Testosteron wurden mit 0,230 ml (0,253 g, 3,238 mMol) Dimethylsulfoxid (DMSO) vermischt, geschüttelt und 30 Minuten stehen gelassen. Es bildete sich eine glasklare Flüssigkeit. Diese wurde weiter mit 0,330 ml (0,294 g, 1,040 mMol) Ölsäure versetzt, geschüttelt und 10 Minuten stehen gelassen. Es bildete sich eine glasklare flüssige Phase. Die monophasische Testosteron-DMSO-Ölsäure-Assoziation löste sich ohne Rückstand in 1,5 ml (1,365 g, 1,542 mMol) Triolein. Es bildete sich eine glasklare flüssige Phase.
Mol-Verhältnis Testosteron:DMSO:Ölsäure = 1:15,6:5

### Beispiel 6: Herstellung einer Lösung von mit DMSO und Ölsäure assoziiertem α-Amyrin in Triolein

### α-Amyrin

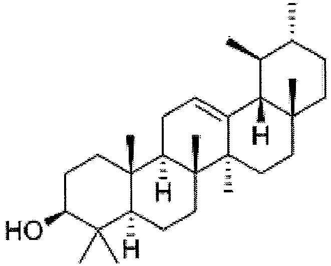

0,003 g (0,007 mMol) α-Amyrin wurden mit 0,06 ml (0,066 g, 0,845 mMol) Dimethylsulfoxid (DMSO) vermischt, geschüttelt und 30 Minuten stehen gelassen. Die α-Amyrin-Kristalle zeigten sich unverändert und nicht gelöst. Diese wurde weiter mit 0,08 ml (0,072 g, 0,253 mMol) Ölsäure versetzt, geschüttelt und 10 Minuten stehen gelassen. Die α-Amyrin-Kristalle lösten sich sofort. Es bildete sich eine glasklare flüssige Phase. Die monophasische α-Amyrin-DMSO-Ölsäure-Assoziation löste sich ohne Rückstand in 0,5 ml (0,910 g, 1,028 mMol) Triolein. Es bildete sich eine glasklare flüssige Phase.
Mol-Verhältnis: α-Amyrin:DMSO:Ölsäure = 1:120:35,8

### Beispiel 7: Herstellung einer Lösung von mit DMSO und Ölsäure assoziierter Salicylsäure in Triolein

### Salicylsäure

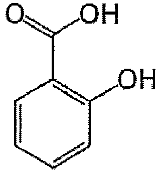

0,125 g (0,905 mMol) Salicylsäure wurden mit 0,386 ml (0,425 g, 5,435 mMol) Dimethylsulfoxid (DMSO) vermischt, geschüttelt und 30 Minuten stehen gelassen. Es bildete sich eine glasklare Flüssigkeit. Diese wurde weiter mit 0,574 ml (0,511 g, 1,809 mMol) Ölsäure versetzt, geschüttelt und 60 Minuten stehen gelassen. Es bildete sich eine glasklare flüssige Phase. Die monophasische Salicylsäure-DMSO-Ölsäure-Assoziation löste sich ohne Rückstand in 1,5 ml (1,365 g, 1,542 mMol) Triolein. Es bildete sich eine glasklare flüssige Phase.
Mol-Verhältnis Salicylsäure:DMSO:Ölsäure = 1:6:2

### Beispiel 8: Herstellung einer Lösung von mit DMSO und Ölsäure assoziiertem Ibuprofen in Triolein

### Ibuprofen

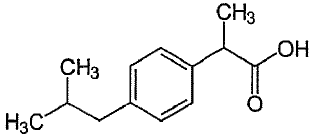

0,125 g (0,606 mMol) Ibuprofen wurden mit 0,043 ml (0,047 g, 0,605 mMol) Dimethylsulfoxid (DMSO) vermischt, geschüttelt und 30 Minuten stehen gelassen. Es bildete sich eine glasklare Flüssigkeit. Diese wurde weiter mit 0,231 ml (0,206 g, 0,728 mMol) Ölsäure versetzt, geschüttelt und 10 Minuten stehen gelassen. Es bildete sich eine glasklare flüssige Phase. Die monophasische Ibuprofen-DMSO-Ölsäure-Assoziation löste sich ohne Rückstand in 1,5 ml (1,365 g, 1,542 mMol) Triolein. Es bildete sich eine glasklare flüssige Phase.
Mol-Verhältnis Ibuprofen:DMSO:Ölsäure = 1:1:1,2

### Beispiel 9: Herstellung einer Lösung von mit DMSO und Ölsäure assoziiertem Ketoprofen in Triolein

### Ketoprofen

| | |
|---|---|
| R-Form | 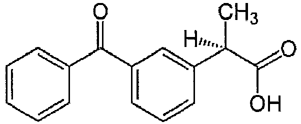 |
| S-Form | 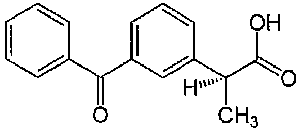 |

0,125 g (0,492 mMol) Ketoprofen wurden mit 0,299 ml (0,229 g, 2,94 mMol) Dimethylsulfoxid (DMSO) vermischt, geschüttelt und 30 Minuten stehen gelassen. Es bildete sich eine glasklare Flüssigkeit. Diese wurde weiter mit 0,499 ml (0,444 g, 1,572 mMol) Ölsäure versetzt, geschüttelt und 10 Minuten stehen gelassen. Es bildete sich eine glasklare flüssige Phase. Die monophasische Ketoprofen-DMSO-Ölsäure-Assoziation löste sich ohne Rückstand in 1,5 ml (1,365 g, 1,542 mMol) Triolein. Es bildete sich eine glasklare flüssige Phase.
Mol-Verhältinis Ketoprofen:DMSO:Ölsäure = 1:6:3,2

### Beispiel 10: Herstellung einer Lösung von mit DMSO und Ölsäure assoziiertem Diclofenac-Natrium in Triolein

### Diclofenac-Na

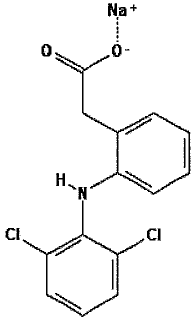

0,06 g (0,189 mMol) Diclofenac-Na wurden mit 0,082 ml (0,090 g, 1,154 mMol) Dimethylsulfoxid (DMSO) vermischt und geschüttelt. Da sich das Diclofenac-Na sehr langsam in DMSO löst, wurde leicht angewärmt und geschüttelt, danach 30 Minuten stehen gelassen. Es bildete sich eine glasklare Flüssigkeit. Diese wurde weiter mit 0,368 ml (0,328 g, 1,160 mMol) Ölsäure versetzt, geschüttelt, leicht erwärmt und dann 30 Minuten stehen gelassen. Es bildete sich eine glasklare flüssige Phase. Die monophasische Diclofenac-Na -DMSO-Ölsäure-Assoziation löste sich ohne Rückstand in 1,5 ml (1,365 g, 1,542 mMol) Triolein. Es bildete sich eine glasklare flüssige Phase.
Mol-Verhältinis Diclofenac-Na:DMSO:Ölsäure = 1: 6,1:6,1

### Beispiel 11: Herstellung einer Lösung von mit DMSO und Ölsäure assoziierter Essigsäure in Triolein

### Essigsäure

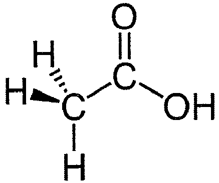

0,105 ml (0,1g, 1,665 mMol) Essigsäure wurden mit 0,120 ml (0,132 g, 1,689 mMol) Dimethylsulfoxid (DMSO) vermischt, geschüttelt und 10 Minuten stehen gelassen. Es bildete sich eine glasklare Flüssigkeit. Diese wurde weiter mit 0,540 ml (0,481 g, 1,701 mMol) Ölsäure versetzt, geschüttelt und 10 Minuten stehen gelassen. Es bildete sich eine glasklare flüssige Phase. Die monophasische Essigsäure -DMSO-Ölsäure-Assoziation löste sich ohne Rückstand in 3,0 ml (2,730 g, 3,083 mMol) Triolein. Es bildete sich eine glasklare flüssige Phase.
Mol-Verhältnis: Essigsäure:DMSO:Ölsäure = 1:1:1

### Beispiel 12: Herstellung einer Lösung von mit DMSO und Ölsäure assoziiertem Calcium-D(+)-pantothenat in Triolein

### Calcium-D(+)-pantothenat

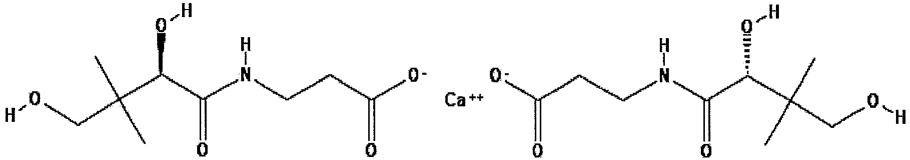

0,22 ml (0,242 g, 3,097 mMol) Dimethylsulfoxid (DMSO) und 0,98 ml (0,872g, 3,088 mMol) Ölsäure wurden vermischt. Es wurden 0,06 g (0,126 mMol) Calcium-D(+)-pantothenat zugegeben, vermischt, leicht erwärmt, geschüttelt und 20 Minuten stehen gelassen. Es bildete sich eine glasklare flüssige Phase (Flüssigkeit 1) ohne Rückstand.

1 ml (0,89 g, 3,151 mMol) Ölsäure und 0,25 ml (0,275 g, 3,520 mMol) Dimethylsulfoxid (DMSO) wurden vermischt, geschüttelt und mit 2,5 ml (2,275 g, 2,569 mMol) Triolein vermischt, geschüttelt und 15 Minuten stehen gelassen. Es bildete sich eine glasklare flüssige Phase (Flüssigkeit 2).

Flüssigkeit (1) und Flüssigkeit (2) wurden vermischt, geschüttelt und 30 Minuten stehenlassen. Es bildete sich eine glasklare flüssige Phase ohne Rückstand.
Mol-Verhältnis Calcium-D(+)-pantothenat:DMSO:Ölsäure = 1:52,5:49,5

### Beispiel 13: Herstellung einer Lösung von mit DMSO und Ölsäure assoziiertem Allantoin in Triolein

### Allantoin

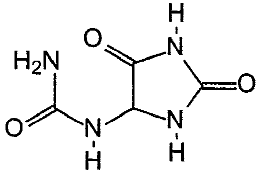

3,0 ml (2,67g, 9,45 mMol) Ölsäure wurden mit 0,671 ml (0,738g, 9,44 mMol) Dimethylsulfoxid (DMSO) vermischt, geschüttelt und 5 Minuten stehen gelassen. Es bildete sich eine glasklare Flüssigkeit (Flüssigkeit 1). 0,041 g (0,266 mMol) Allantoin wurden mit Flüssigkeit 1 vermischt, 30 Minuten geschüttelt. Es bildete sich eine milchige Flüssigkeit. Dies wurde weiter mit 1,05 ml (1,16g, 14,78 mMol) Dimethylsulfoxid (DMSO) vermischt, geschüttelt, leicht erwärmt und 30 Minuten stehen gelassen. Es bildete sich eine glasklare flüssige Phase (monophasische Allantoin-DMSO-Ölsäure-Assoziation).

5,67 ml (5,04 g, 17,87 mMol) Ölsäure und 2,36 ml (2,60 g, 33,23 mMol) Dimethylsulfoxid (DMSO) wurden vermischt, geschüttelt und mit 9,44 ml (8,59 g, 9,70 mMol) Triolein vermischt, geschüttelt und 15 Minuten stehen gelassen. Es bildete sich eine glasklare Flüssigkeit (Flüssigkeit 2).

Die monophasische Allantoin-DMSO-Ölsäure-Assoziation löste sich ohne Rückstand Flüssigkeit 2. Es bildete sich eine glasklare flüssige Phase.
Mol-Verhältnis Allantoin:DMSO:Ölsäure = 1:216.3:102,8

Die folgenden Bezugs-Beispiele 14 und 15 betreffen chemische Verbindungen, die keine pharmazeutischen Wirkstoffe sind, werden aber bereitgestellt, um strukturelle Voraussetzungen für die Löslichkeit zu erläutern.

### Bezugs-Beispiel 14: Herstellung einer Lösung von mit DMSO und Ölsäure assoziiertem Cumarin in Triolein

### Cumarin

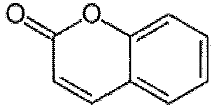

0,100 g (0,684 mMol) Cumarin wurden mit 0,097 ml (0,107 g, 1,366 mMol) Dimethylsulfoxid (DMSO) vermischt, geschüttelt und 10 Minuten stehen gelassen. Es bildete sich eine glasklare Flüssigkeit. Diese wurde weiter mit 0,652 ml (0,580 g, 2,054 mMol) Ölsäure versetzt, geschüttelt und 60 Minuten stehen gelassen. Es bildete sich eine glasklare flüssige Phase. Die monophasische Cumarin-DMSO-Ölsäure-Assoziation löste sich ohne Rückstand in 1,5 ml (1,365 g, 1,542 mMol) Triolein. Es bildete sich eine glasklare flüssige Phase.
Mol-Verhältnis Cumarin:DMSO:Ölsäure = 1:2:3

### Bezugs-Beispiel 15: Herstellung einer Lösung von mit DMSO und Ölsäure assoziiertem Imidazol in Triolein

### Imidazol

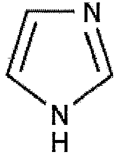

0,125 g (1,836 mMol) Imidazol wurden mit 0,156 ml (0,172 g, 2,196 mMol) Dimethylsulfoxid (DMSO) vermischt, geschüttelt und 30 Minuten stehen gelassen. Es bildete sich eine glasklare Flüssigkeit. Diese wurde weiter mit 0,698 ml (0,621 g, 2,199 mMol) Ölsäure versetzt, geschüttelt und 10 Minuten stehen gelassen. Es bildete sich eine glasklare Phase. Die monophasische Imidazol-DMSO-Ölsäure-Assoziation löste sich ohne Rückstand in 1,5 ml (1,365 g, 1,542 mMol) Triolein. Es bildete sich eine glasklare flüssige Phase.
Mol-Verhältnis Imidazol:DMSO:Ölsäure = 1:1,2:1,2

### Beispiel 16: Absorption der in Beispiel 1 hergestellten Triolein-Lösung der Curcumin-DMSO-Ölsäure-Assoziation durch die Haut

Die in Beispiel 1 hergestellt Lösung wurde auf die Haut des Handrückens aufgetragen. Nach 1 Sunde war nach Entfernen überschüssiger Öllösung von der Haut noch ein gelber Fleck in der Haut zu erkennen, der von in oberflächliche Hautbereiche absorbiertem Curcurmin herrührte. Nach 1 Stunde war der gelbe Fleck völlig verschwunden, was bedeutet, dass das Curcumin völlig durch die Haut penetriert war.

## Patentansprüche

1. Topische kosmetische und/oder pharmazeutische Zusammensetzung, bestehend aus einer hydrophobe Ölphase, wobei die hydrophobe Ölphase Dimethylsulfoxid, eine oder mehrere bei Raumtemperatur flüssige Monocarbonsäuren mit mindestens 6 Kohlenstoffatomen und mindestens einen Wirkstoff gelöst enthält, welcher einen ungesättigten und/oder aromatischen Ring und/oder eine Wasserstoffbrücken bildende Gruppe enthält, wobei das Mol-Verhältnis von Wirkstoff(en) : Dimethylsulfoxid : Monocarbonsäure(n) im Bereich von 1 : 1 - 700 : 1- 180 liegt und das Mol-Verhältnis von Dimethylsulfoxid :
Monocarbonsäure im Bereich von 1 : 5 bis 12 : 1 liegt und eine Mischung bestehend aus Dimethylsulfoxid, Monocarbonsäure und Wirkstoff, wie vorstehend definiert, eine klare Phase bilden kann,
und wobei die Ölphase weiter mindestens ein Triglycerid oder natürliches Triglyceridöl umfasst.

2. Topische kosmetische und/oder pharmazeutische Zusammensetzung nach Anspruch 1, **dadurch gekennzeichnet, dass** die flüssige Monocarbonsäuren oder Fettsäure Ölsäure ist.

3. Topische kosmetische und/oder pharmazeutische Zusammensetzung nach Anspruch 1 oder 2, **dadurch gekennzeichnet, dass** der Wirkstoff aus α-Amyrin, β-Amyrin, 3α-O-Acetyl-β-boswelliasäure, 3α-O-Acetyl-11-keto-β-boswelliasäure, 3α-O-Acetyl-11-hydroxy-β-boswelliasäure, 3α-O-Acetyl-9,11-dehydro-β-boswelliasäure, 3α-O-Acetyl-11-methoxy-β-boswelliasäure, 3β-Hydroxy-β-boswelliasäure, 11α-Hydroxy-β-boswelliasäure, 11β-Hydroxy-β-boswelliasäure, 11-Keto-ß-boswelliasäure, β-Boswelliasäure, 3-O-Galloyl-β-boswelliasäure, 3-O-Galloyl-11-keto-β-boswelliasäure, Oleanolsäure, Ursolsäure, Maslinsäure, Echinocystsäure, Lupansäure, Ferulasäure, 3α-O-Acetyllupansäure, 3α-O-Acetyl-28β-hydroxylupansäure, Robursäure, 4(23)-Dihydroketorobursäure, Bisabolol, (-)-α-Bisabolol, β-Bisabolol, Sulforaphan, Curcumin, Bisdesmethoxycurcumin, Quercetin, Luteolin, Apigenin, Umckalin, Scopoletin, Helenalin, 11,12-Dihydrohelenalin, Kämpferol, Kämpferid, Rhamnetin, β-Sitosterol, Allantoin, Epigallocatechingallat, Coffein, Testosteron, Progesteron, Ibuprofen, Ketoprofen, Benzoesäure, Acetylsalicylsäure, Salicylsäure, Diclofenac-Na, Diclofenac, Calcium-D(+)-pantothenat und Pantothensäure ausgewählt ist.

## Claims

1. Topical cosmetic and/or pharmaceutical composition consisting of a hydrophobic oil phase, wherein the hydrophobic oil phase contains dimethyl sulfoxide, one or more monocarboxylic acids which are liquid at room temperature and have at least 6 carbon atoms, and at least one active ingredient in solution, which active ingredient contains an unsaturated and/or aromatic ring and/or a group forming hydrogen bridges, the molar ratio of active ingredient(s): dimethyl sulfoxide:
monocarboxylic acid(s) being in the range of 1 : 1-700 : 1-180 and the molar ratio of dimethyl sulfoxide : monocarboxylic acid is in the range of 1 : 5 to 12 : 1, and a mixture consisting of dimethyl sulfoxide, monocarboxylic acid, and active ingredient, as defined above, can form a clear phase,
and wherein the oil phase further comprises a triglyceride or a natural triglyceride oil.

2. Topical cosmetic and/or pharmaceutical composition according to claim 1, **characterized in that** the monocarboxylic or fatty acid is oleic acid.

3. Topical cosmetic and/or pharmaceutical composition according to claim 1 or 2, **characterized in that** the active ingredient is selected from α-amyrin, β-amyrin, 3α-O-acetyl-β-boswellic acid, 3a-O-acetyl-11-keto β-boswellic acid, 3α-O-acetyl-11-hydroxy-p-boswellic acid, 3α-O-acetyl-11-hydroxy-β-boswellic acid, 3a-O-acetyl-9,11-dehydro-β-boswellic acid, 3α-O-acetyl-11-methoxy-β-boswellic acid, 3β-hydroxy-β-boswellic acid, 11α-hydroxy-β-boswellic acid, 11-β-hydroxy-β-boswellic acid, 11-keto-β-boswellic acid, β-boswellic acid, 3-O-galloyl-β-boswellic acid, oleanolic acid, ursolic acid, maslinic acid, echinocystic acid, lupanic acid, ferulic acid, 3α-O-acetyllupanoic acid, 3α-O-acetyl-28β-hydroxylupanoic acid, roburic acid, 4(23)-dihydroketoroburic acid, bisabolol, (-)-α-bisabolol, β-bisabolol, sulforaphane, curcumin, bisdesmethoxycurcumin, quercetin, luteolin, apigenin, umckalin, scopoletin, helenalin, 11,12-dihydrohelenalin, kaempferol, kaempferid, rhamnetin, β-sitosterol, allantoin, epigallocatechin gallate, caffeine, testosterone, progesterone, ibuprofen, ketoprofen, benzoic acid, acetylsalicylic acid, salicylic acid, diclofenac-Na, diclofenac, calcium-D (+)-pantothenate and pantothenic acid.

## Revendications

1. Composition cosmétique et/ou pharmaceutique topique consistant en une phase huileuse hydrophobe contenant du diméthylsulfoxyde, un ou plusieurs acide(s) monocarboxylique(s) liquide(s) à température ambiante et ayant au moins 6 atomes de carbone et au moins un principe actif en solution, le principe actif contenant un cycle insaturé et/ou aromatique et/ou un groupe formant des liaisons hydrogènes, le rapport molaire principe(s) actif (s): diméthylsulfoxyde: acide(s) monocarboxylique(s) étant dans la gamme de 1: 1-700: 1-180 et le rapport molaire de diméthylsulfoxyde:
acide monocarboxylique étant dans la gamme de 1 : 5 à 12 : 1 et un mélange consistant en diméthylsulfoxyde, acide monocarboxylique et principe actif, tel que défini précédemment, étant capable de former une phase claire,
la phase huileuse comprenant en outre au moins un triglycéride ou une huile triglycéridique naturelle.

2. Composition cosmétique et / ou pharmaceutique topique selon la revendication 1, **caractérisée en ce que** l'acide monocarboxylique ou l'acide gras liquide est l'acide oléique.

3. Composition topique cosmétique et/ou pharmaceutique selon revendication 1 ou 2, **caractérisée en ce que** le principe actif est choisi parmi l'a-amyrine, la β-amyrine, l'acide 3α-O-acétyl-β-boswellique, l'acide 3α-O-acétyl-11-céto β-boswellique, l'acide 3α-O-acétyl-11-hydroxy-β-boswellique, l'acide 3α-O-acétyl-9,11-déhydro-β-boswellique, l'acide 3α-O-acétyl-11-méthoxy-β-boswellique, l'acide 3β-hydroxy-β-boswellique, l'acide 11α-hydroxy-β-boswellique, l'acide 11β-hydroxy-β-boswellique, l'acide 11-céto-β-boswellique, l'acide β-boswellique, l'acide 3-O-galloyl-β-boswellique, l'acide 3-O-galloyl-11-céto-β-boswellique, l'acide oléanolique, l'acide ursolique, l'acide maslinique, l'acide échinocystique, l'acide lupanique, l'acide férulique, l'acide 3α-O-acétyllupanoïque, l'acide 3α-O-acétyl-28β-hydroxylupanoïque, l'acide roburique, l'acide 4(23)-dihydrokétoroburique, le bisabolol, le (-) -α-bisabolol, le β-bisabolol, le sulforaphane, le curcumine, le bisdesméthoxycurcumine, le quercétine, le lutéoline, l'apigénine, l'umckaline, le scopolétine, l'hélénaline, le 11,12-dihydroxyhélénaline, le kaempférol, le kaempféride, le rhamnetine, le β-sitostérol, l'allantoïne, le gallate d'épigallocatéchine, la caféine, la testostérone, la progestérone, l'ibuprofène, le kétoprofène, l'acide benzoïque, l'acide acétylsalicylique, l'acide salicylique, le diclofénac-Na, le diclofénac, le calcium-D (+) - pantothénate et l'acide pantothénique.
